# EUROPEAN PATENT APPLICATION

(11) **EP 4 407 024 A1**
(43) Date of publication of application: **31.07.2024**
(21) Application number: 23153832.3
(22) Date of filing: 28.01.2023
(51) Int. Cl.: C12N 5/0786, A61K 35/15

(54) **EXTRACELLULAR VESICLES FROM REGULATORY MACROPHAGES**

(71) Applicant: Trizell GmbH, 20354 Hamburg (DE)
(72) Inventor: ALBRECHT, Martin, 24414 Kiel (DE); ZITTA, Karina, 24103 Kiel (DE); BERND, Rouven, 24103 Kiel (DE); FÄNDRICH, Fred, 24105 Kiel (DE)
(74) Representative: Uexküll & Stolberg

(57) **Abstract**

The present invention relates to extracellular vesicles which are derived from immunoregulatory macrophage cells. The vesicles exert immunoregulatory, angiogenic and tissue regenerative properties and are thus useful for the treatment of different diseases and conditions. In a further aspect, the invention relates to a pharmaceutical composition comprising the extracellular vesicles of the invention. In a still further aspect, the invention relates to the use of the extracellular vesicles or a pharmaceutical composition comprising such vesicles in medicine. In a still further aspect, the invention relates to the use of the extracellular vesicles or a pharmaceutical composition comprising such vesicles for treating or preventing immunological and non-immunological diseases and conditions, including autoimmune diseases, inflammatory diseases, hypersensitivity reactions, transplant rejection and micro- or macroangiopathies of the lower limbs. The invention also provides a process for preparing the macrophage-derived extracellular vesicles from blood monocytes. Finally, the invention provides a process for preparing immunoregulatory T cells which makes use of the extracellular vesicles.

## Description

### FIELD OF THE INVENTION

The present invention relates to extracellular vesicles which are derived from immunoregulatory macrophage cells. The vesicles exert immunoregulatory, angiogenic, anti-inflammatory and tissue regenerative properties and are thus useful for the treatment of different diseases and conditions. In a further aspect, the invention relates to a pharmaceutical composition comprising the extracellular vesicles of the invention. In a still further aspect, the invention relates to the use of the extracellular vesicles or a pharmaceutical composition comprising such vesicles in medicine. In a still further aspect, the invention relates to the use of the extracellular vesicles or a pharmaceutical composition comprising such vesicles for treating or preventing immunological and non-immunological diseases and conditions, including autoimmune diseases, inflammatory diseases, hypersensitivity reactions, transplant rejection and micro- or macroangiopathies. The invention also provides a process for preparing the macrophage-derived extracellular vesicles from blood monocytes. Finally, the invention provides a process for preparing immunoregulatory T cells which makes use of the extracellular vesicles.

### BACKGROUND

Transferring immunoregulatory cells from a tolerant donor to non-tolerant recipient as a means of establishing tolerance in the recipient is a well-known technique in experimental immunology, but its clinical application is only now receiving serious attention [1]. At present, several immunoregulatory cell types are reaching the point of preclinical development, which will allow them to be investigated as immunosuppressive agents in early-phase clinical trials, including regulatory T cells [2], tolerogenic dendritic cells [3] and regulatory macrophages [4].

A broad spectrum of immunologic conditions may be amenable to treatment with cell-based immunoregulatory therapies, including T cell- and B cell-mediated autoimmune disease, chronic inflammatory disorders, graft-versus-host disease (GVHD), and transplant rejection. In these conditions, cell-based immunoregulatory therapies might reduce or even obviate the need for general immunosuppressive or anti-inflammatory therapy, thereby sparing patients its attendant complications. Because the kind of immunologic tolerance supported by regulatory cells is dominant and self-sustaining, there exists the possibility that cell-based immunotherapy may offer a curative option in diseases that would otherwise require long-term general immunosuppressive or anti-inflammatory therapy.

One particularly promising candidate cell type for use as an adjunct immunosuppressive agent in transplantation is the immunoregulatory macrophage (referred to herein and in the literature as "Mreg"). The Mreg cell reflects a unique state of macrophage differentiation, which is distinguished from macrophages in other activation states by its robust phenotype and potent T-cell suppressor function [5]. Human Mregs potently suppress mitogen-stimulated T-cell proliferation in vitro, which can be attributed to interferon (IFN) γ-induced indoleamine 2,3-dioxygenase activity, as well as contact-dependent deletion of activated T cells. In addition, Mregs drive the development of activated induced regulatory T cells that, in turn, suppress the proliferation of effector T cells and inhibit the maturation of dendritic cells. Therefore, when Mregs are administered to a recipient, it is hypothesized that a feed-forward loop of immunologic regulation is initiated leading to the long-term immunologic acceptance of a foreign transplant or prevention of immunopathology. Mreg-containing cell preparations have been administered to kidney transplant recipients as a form of adjunct immunosuppressive treatment in a series of case studies and two early-phase clinical trials [5]-[9]. These pilot studies clearly demonstrate the feasibility of this technique for solid organ transplantation.

Despite the great progress that was made in recent years in the field of immunoregulatory cells, there are still concerns about safety of cell therapy. In particular, the ability of cells to change into inappropriate cell types, such as cancer cells, is a persisting challenge to patient safety. Accordingly, there is an ongoing need for immunoregulatory cells or agents that can be used for therapeutic purposes, e.g. for inducing immunologic acceptance of a foreign transplant in a recipient or for treating different types of diseases, which exert a reduced safety risk.

### DESCRIPTION OF THE INVENTION

EVs represent a heterogeneous group of membrane-surrounded structures that are naturally released from most cells. They typically contain lipids, proteins and RNAs and represent an efficient way to transfer functional cargos from cell to cell [10, 11]. Recent literature also supports a role for EV in mediating complex and coordinated communication among different cell types [12, 13, 14].

The invention is based on the insight that Mreg cells prepared in accordance with established protocols produce extracellular vesicles (EV) which exert immunoregulatory, angiogenic, anti-inflammatory and tissue regenerative properties that correspond to those of the Mreg cell from which they are derived. Accordingly, the EV can be isolated and used as active ingredients for treating or preventing a number of different disorders and diseases. Due to their inability to multiply, the vesicles do not involve the same safety concerns as the cells from which they have been derived. Specifically, the Mreg-derived vesicles are immunoregulatory and anti-inflammatory which means that they can suppress unwanted T cell responses by inhibiting T cell expansion. This can be tested as described below in Example 7. The vesicles are moreover angiogenic which means that they promote the formation of new blood vessels, e.g. in hypoxic tissues. The ability of forming new blood vessels can be tested in the tube formation assay described below in Example 6. Apart from that, the vesicles are tissue-regenerative which means that they are able to repair damaged tissue, e.g. in wounds and ulcers. The ability of regenerating damaged tissue can be tested in the scratch assay described below in Example 6.

Therefore, in a first aspect the present invention relates to macrophage-derived extracellular vesicles having a size between 0.1-10 µm and exerting an immunoregulatory activity. The size of the vesicles is preferably analyzed using a coulter counter, such as a MOXI counter. Preferably, the macrophage-derived vesicles of the invention have a size of between 1-10 µm which means that they belong to the group of so-called "large extracellular vesicles" or "L-EVs". In practice, the vesicles of the invention will typically be provided as a population of vesicles of different sizes. In these cases, it will be preferred that at least 60%, at least 70%, at least 80%, at least 90%, or at least 95% of the vesicles in the population are L-EVs, i.e. have a 1-10 µm.

The macrophage-derived extracellular vesicle does preferably not contain a nucleus. In addition, the vesicle does contain no more than traces of double-stranded DNA. Preferably, the vesicle is free of double-stranded DNA. The presence of double-stranded DNA can be measured photospectrometrically or by gel electrophoresis. In one preferred embodiment, the presence of double-stranded DNA is measured photospectrometrically. In another preferred embodiment, the presence of double-stranded DNA is measured by gel electrophoresis. More preferably, the vesicles of the invention do not contain any cell organelle selected from the group of nucleus, mitochondria, endoplasmatic reticulum and Golgi complex. While the vesicles may contain fragments of such organelles, it is preferred that they do not encompass complete functional organelles.

On the other hand, the vesicle may contain different proteins, lipids and RNAs as functional cargo. In a preferred embodiment, the macrophage-derived extracellular vesicles comprise one or more proteins which induce angiogenesis, such as interleukin-8 and angiogenin. In one embodiment, the vesicles of the invention contain one or more protein selected from the group of interleukin-8, platelet factor-4, serpin E1, serpin F1, TIMP-1, angiogenin, chemo-kine ligand 16 (CXCL16), dipeptidyl peptidase IV (DPPIV), endoglin (ENG), insulin-like growth factor binding protein 2 (IGFBP-2), insulin-like growth factor binding protein 3 (IGFBP-3), matrix metalloproteinase-8 (MMP-8), matrix metalloproteinase-9 (MMP-9), thrombospondin 1 (THBS1), and human plasminogen activator, urokinase (uPA). In yet another embodiment, the vesicles of the invention contain one or more protein selected from the group of interleukin-8, platelet factor-4, serpin E1, serpin F1, TIMP-1, and angiogenin. Preferably, these proteins are human proteins.

In another embodiment, the vesicles of the invention contain 2, 3, 4, or 5 of these proteins. In a most preferred embodiment, the vesicles of the invention contain all of these proteins. These proteins are also produced and secreted de novo when Mreg are cultured for 24h after harvest [15]. With regard to angiogenesis, looking at the proteins factors individually does not provide a uniform picture: interleukin-8 and angiogenin are potent stimulators of angiogenesis [16, 17], while platelet factor-4 is mainly known as angiogenesis inhibitor [18]. Serpin E1 (PAI-1) appears to exert pro- or antiangiogenic functions [19] and the ubiquitous and multifunctional proteins serpin F1 and TIMP-1 show as well ambivalent functions regarding angiogenesis [20, 21, 22]. However, it can be assumed that not a single factor but rather the combination of intravesicular factors together with the membrane-bound CD molecules and receptor composition on or within the target cells (i.e. endothelial cells) is responsible for the observed pro-angiogenic effects described in the below working examples.

The extracellular vesicles of the invention comprise surface markers that have been described earlier in connection with Mreg cells. Preferably, the vesicles of the invention comprise one or more markers selected from the group consisting of CD11c, CD86, CD31 and CD45 on its surface. At the same time, the vesicles preferably also comprise also one or more markers which are typically found on extracellular vesicles, such as LAMP-1, CD9, CD63 and CD81 on its surface. Thus, in a preferred embodiment, the vesicles of the invention comprise one of the following marker patters on its surface:
(1) CD11c, CD86, CD31;
(2) CD11c, CD86, CD31, LAMP-1;
(3) CD11c, CD86, CD31, LAMP-1, CD9;
(4) CD11c, CD86, CD31, LAMP-1 CD9, CD63;
(5) CD11c, CD86, CD31, LAMP-1 CD9, CD63, CD81;
(6) CD11c, CD86, CD45;
(7) CD11c, CD86, CD45, LAMP-1;
(8) CD11c, CD86, CD45, LAMP-1, CD9;
(9) CD11c, CD86, CD45, LAMP-1 CD9, CD63;
(10) CD11c, CD86, CD45, LAMP-1 CD9, CD63, CD81;
(11) CD11c, CD31, CD45;
(12) CD11c, CD31, CD45, LAMP-1;
(13) CD11c, CD31, CD45, LAMP-1, CD9;
(14) CD11c, CD31, CD45, LAMP-1 CD9, CD63;
(15) CD11c, CD31, CD45, LAMP-1 CD9, CD63, CD81;
(16) CD86, CD31, CD45;
(17) CD86, CD31, CD45, LAMP-1;
(18) CD86, CD31, CD45, LAMP-1, CD9;
(19) CD86, CD31, CD45, LAMP-1 CD9, CD63; or
(20) CD86, CD31, CD45, LAMP-1 CD9, CD63, CD81.

The detection of the above surface markers can be achieved by different routine methods. Preferably, the detection of surface markers on the vesicles of the invention involves flow cytometry. Flow cytometry is a widely used method for analyzing the expression of cell surface markers and intracellular molecules. It is routinely used for applications like cell counting, cell sorting, and biomarker profiling. In particular, it can be used for defining different cell types in a heterogeneous cell population. Flow cytometry typically measures the fluorescence intensity produced by fluorescent-labeled antibodies that detect markers on a cell or particle surface. In a particularly preferred embodiment, the vesicles of the invention exert one of the above marker patterns as determined by flow cytometry. The invention preferably pertains to compositions, such as pharmaceutical compositions, comprising a population of vesicles, in which at least 50% of the vesicles, and preferably at least 60% of the vesicles, at least 70% of the vesicles, at least 80% of the vesicles, at least 90% of the vesicles, or at least 95% of the vesicles, exert one of the above marker patterns.

The invention also pertains to macrophage-derived extracellular vesicles which are obtainable by a method which includes, as a first step, the differentiation of CD14 positive blood monocytes into Mregs by culturing the monocytes in a culture medium in the presence of (i) M-CSF and/or GM-CSF, (ii) a CD16 ligand and (iii) IFN-y and, as a second step, the obtainment of the extracellular vesicles from the culture medium.

For therapeutic use, the Mreg-derived extracellular vesicles of the invention will be formulated as a pharmaceutical composition. Therefore, in a second aspect the invention relates to a pharmaceutical composition comprising the macrophage-derived extracellular vesicles according to the first aspect of the invention. The pharmaceutical composition will comprise, as a first component, an effective amount of macrophage-derived extracellular vesicles, and preferably Mreg-derived extracellular vesicles.

As used herein, an effective amount of vesicles to be administered to the patient will be in the range of about 1×10⁴ to about 1×10⁹/kg body weight, preferably between about 1×10⁴ to about 1×10⁸/kg body weight, more preferably between about 1×10⁵ and about 1×10⁷/kg body weight, and even more preferably between about 1×10⁶ and about 1×10⁷/kg body weight, such as about 1× 10⁶/kg, about 2×10⁶/kg, about 3×10⁶/kg, about 4×10⁶/kg, about 5×10⁶/kg, about 6×10⁶/kg, about 7×10⁶/kg, or about 8×10⁶/kg body weight of the patient to be treated.

Apart from the extracellular vesicles, the pharmaceutical composition can comprise further excipients, such as buffers, pH regulating agents, preservatives, and the like. The nature and amounts of the excipients included into the pharmaceutical composition will depend on the intended route of administration.

Generally, different routes of administration are feasible for providing the macrophage-derived extracellular vesicles to a patient in need of treatment. Preferably, the pharmaceutical composition of the invention will be formulated for parenteral administration, such as subcutaneous, intramuscular, intravenous or intradermal administration. In one embodiment, the extracellular vesicles of the invention or a composition comprising said vesicles are administered to the patient by intravenous administration, e.g. by injection or infusion. Pharmaceutical compositions suitable for intravenous administration by injection or infusion normally include sterile aqueous solutions or suspensions and sterile powders for the extemporaneous preparation of sterile solutions or suspensions. The formulation of the macrophage-derived extracellular vesicles into pharmaceutical compositions can be achieved by applying routine methods known in the field of drug formulation. Suitable methods are described, for example, in standard textbooks.

For administration by injection or infusion, suitable carriers may comprise physiological saline, bacteriostatic water, Cremophor EL^{™} (BASF) or phosphate buffered saline (PBS). The carrier may also be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Sterile injectable solutions can be prepared by incorporating the vesicles of the invention in the required amount in an appropriate solvent with one or more of the above mentioned ingredients followed by sterile filtration. Generally, suspensions are prepared by incorporating the active compound, i.e. the macrophage-derived extracellular vesicles, into a sterile vehicle that contains a basic dispersion medium and the required other ingredients from those mentioned above. The pharmaceutical composition should be stable upon administration and is preferably protected from contamination with microorganisms, such as bacteria and fungi, by including parabens, chlorobutanol, phenol, ascorbic acid, thimerosal, or the like into the composition.

Where the pharmaceutical composition is intended for injection, the total volume to be injected is between 1 and 100 ml, preferably between 10 and 50 ml, such as 20 ml, 30 ml or 40 ml. Where the pharmaceutical composition is intended for infusion, the total volume to be infused will be between 50 and 500 ml, wherein a volume of between 90 ml and 250 ml is particularly preferred, and wherein a volume of between 90 ml and 150 ml is even more preferred.

The macrophage-derived vesicles can be administered to a patient in need of treatment by different administration regimens. For example, where the macrophage-derived vesicles are administered to the patient by intravenous infusion, the total amount of vesicles to be administered can be supplied by one or more than one infusion. The suspension comprising the macrophage-derived vesicles may be primed with 0.9% NaCl. The suspension also may be given in a single infusion, more preferably a short-term infusion within less than 60 min, e.g. within 60 min, within 30 min, within 20 min or within 15 min or less. Preferably, a central venous catheter is used for administering the macrophage-derived extracellular vesicle suspension.

The administration of the macrophage-derived vesicles can be accompanied by the preceding, simultaneous or subsequent administration of other active agents. For example, where the vesicles are administered to patients suffering from micro- or macroangiopathies, compounds against blood hyperviscosity, such as calcium dobesilate, or compounds which exert a capillary stabilizing effect, such as naftazone, can be administered before, simultaneously with or after vesicle administration.

For safety reasons, it is recommended that the macrophage-derived vesicles of the invention are administered within 24 hours after harvesting them from the cell cultures. Preferably, the vesicles are administered within 20 hours, within 16 hours, within 12 hours, within 8 hours, or within 4 hours after harvesting the cells from the cultures.

In a third aspect, the invention relates to a process for preparing the macrophage-derived extracellular vesicles of the invention having immunoregulatory, angiogenic, anti-inflammatory and tissue regenerative properties. It has been found that these vesicles are produced during the conversion of monocytes into immunoregulatory macrophages (Mregs). The preparation of Mregs has been extensively described in the literature. Mregs are derived from human CD14+ blood monocytes. In order to induce the characterizing biological properties of Mregs, the monocytes are treated with a specific combination of growth factors, cytokines and receptor ligands. The cells obtained from this process are characterized by a unique phenotype that distinguishes them from blood monocytes, monocyte-derived dendritic cells and other suppressive myelomonocytic cell products.

Accordingly, the invention relates to a process for preparing the macrophage-derived extracellular vesicles having immunoregulatory activity, said process comprising:
(a) isolating CD14 positive monocytes from a blood sample of a subject;
(b) culturing the monocytes in a culture medium containing (i) M-CSF and/or GM-CSF, and (ii) a CD16 ligand;
(c) contacting the cells with IFN-y; and
(d) obtaining the macrophage-derived extracellular vesicles from the culture medium.

After performing steps (a)-(c), the culture medium will comprise both immunoregulatory Mregs and immunoregulatory Mreg-derived extracellular vesicles. Therefore, the separation of the vesicles from the culture medium in step (d) can be performed in two steps. In a first step (d1) the Mregs are separated from the medium, e.g. by centrifugation at low gravitational force, e.g. at 300-400xg. The cells will be present in the sediment after centrifugation, while the vesicles are still in the supernatant. To harvest the vesicles, the supernatant is then subjected in a second step (d2) to centrifugation at high gravitational force, e.g. at 3000-4000xg. After the centrifugation at high gravitational force, the vesicles will be present in the sediment and can be easily separated from the culture medium.

The above method for preparing the macrophage-derived extracellular vesicles uses blood monocytes as starting material. While it will be preferred that human blood monocytes are used, the method is not restricted to the use of cells of human origin. In fact, it is applicable also to other types of non-human cells, in particular vertebrate cells, e.g. non-human primate or pig cells. It is however preferred that the monocytes which serve as a starting material are obtained from the blood of a human donor, and more preferably from the peripheral blood of a human donor. The donor may be a healthy subject or a patient suffering from one or more diseases. The monocyte donor may be the intended recipient of the vesicles obtained from the differentiated Mreg cells (autologous approach). Alternatively, the monocyte donor may be a separate person from the intended recipient of the vesicles obtained from the differentiated Mreg cells (allogeneic approach). In the latter case, the donor and recipient may be genetically related or unrelated. The preferred relationship between donor and recipient depends upon the intended clinical application. The use of vesicles obtained from autologous Mreg cells may help to avoid certain adverse reactions. Therefore, the use of vesicles obtained from autologous Mreg cells is preferred.

Different methods are known in the art for the enrichment of monocytes from blood, and each of these methods can be used in the context with the above preparation method. For example, blood obtained by venepuncture can be treated with an anticoagulant and subsequently separated by use of a separation medium, such as Ficoll-Paque Plus. For this, the anticoagulant-treated blood sample is layered on the Ficoll-Paque Plus solution and centrifuged, which will result in the formation of layers containing the different cell types. The bottom layer contains erythrocytes which have been aggregated and sedimented by the Ficoll-Paque Plus reagent. The layer immediately above the erythrocyte layer contains mostly granulocytes which have migrated through the Ficoll-Paque Plus layer. Owing to their lower density, monocytes and lymphocytes are found at the interface between the plasma and the Ficoll-Paque Plus. Enrichment of the mononuclear cell fraction can be achieved by isolation of the layer and subsequent washing and centrifugation.

Another routinely used method for separating mononuclear leucocytes from blood samples includes leukapheresis. Leukapheresis is a specific type of apheresis in which white blood cells are obtained from peripheral blood according to their relative densities in a continuous process. In this procedure, the blood of a subject is passed through a special centrifugation device which collects the chosen fraction of white blood cells and returns the remaining blood cells and plasma back to the donor. Leukapheresis is nowadays a routine clinical measure for obtaining leucocytes or stem cells from peripheral blood. Different devices are available from several manufactures that can be used for performing leukapheresis in the context with the present invention, e.g. the Spectra Optia^{®} Apheresis System from Terumo BCT. Where the leukapheresis is carried out by use of the COBE^{®} Spectra Apheresis System, it is preferred to use the manual protocol provided by the manufacturer, since this protocol was found to result in better quality monocytes compared to the AutoPBSC protocol.

Both the use of a separation medium like Ficoll-Paque Plus and the use of a leukapheresis device will provide a cell fraction that contains, apart from the monocytes, also lymphocytes. According to the invention, monocytes may be enriched and separated from the lymphocytes by known methods, e.g. by magnetic bead separation, elutriation, filtration or plastic adherence, before the cells are used for Mreg and vesicle preparation. However, it is not mandatory to use a homogeneous monocyte fraction for Mreg preparation. In fact, the presence of an amount of 0.1-20%, preferably 10-20% lymphocytes in the monocyte fraction may positively influence the differentiation of monocytes to Mregs.

For obtaining a mononuclear cell preparation that is enriched for monocytes, peripheral blood mononuclear cells may be contacted, e.g., with CD14 microbeads to which CD14-positive monocytes bind. The monocytes in step (a) of the above method may be isolated by leukapheresis and subsequently subjected to separation step using CD14 affinity molecules, preferably CD14 antibodies. Such a purification step massively reduces contamination of the starting material with non□monocytes. The isolation of CD14 monocytes can be achieved by automated systems. For example, the CD14 monocytes to be used in the above method can be isolated with the CliniMACS^{®} Technology (Miltenyi Biotec GmbH, Bergisch Gladbach, Germany).

The monocyte fraction, which has been isolated by leukapheresis and/or other methods, can directly be used for differentiation of the cells by incubation with M-CSF and/or GM-CSF and the CD16 ligand, or it can be stored in autologous plasma supplemented with Anticoagulant Citrate Dextrose Solution (ACD-A) or any other suitable buffer until further use, e.g. overnight at 4°C. If the isolated monocyte fraction has to be transported to a different site where the differentiation process is carried out, care should be taken that differentiation of the cells by incubation with M-CSF/GM-CSF is started within 24 hours after isolation of the cells, preferably within 18 hours, within 12 hours, within 6 hours, within 4 hours, or within 2 hours after isolation of the monocytes. For long term storage, the monocyte fraction may be resuspended in a suitable cryopreservation solution and stored at temperatures below 20°C, preferably below 80°C for extended periods of time.

After isolation of the monocytes, the cells are incubated in the presence of Macrophage Colony-Stimulating Factor (M-CSF, also known as CSF1), Granulocyte Macrophage Colony-Stimulating factor (GM-CSF), or both, and a CD16 ligand. M-CSF is known in the art as a hematopoietic growth factor that influences the proliferation, differentiation, and survival of monocytes, macrophages, and bone marrow progenitor cells. Granulocyte-macrophage colony-stimulating factor (GM-CSF, also known as CSF2), is a monomeric glycoprotein that functions as a cytokine and is secreted by macrophages, T cells, mast cells, NK cells, endothelial cells and fibroblasts. M-CSF and GM-CSF proteins from different species have been described and can be purchased from different manufacturers. The choice of the M-CSF and/or GM-CSF will depend on the origin of the monocytes which are to be differentiated into Mreg cells. For example, if human monocytes are differentiated to Mregs using the process described above, the medium used will contain human M-CSF and/or human GM-CSF, preferably recombinant human M-CSF and/or recombinant human GM-CSF. Similarly, if porcine monocytes are used in the differentiation method, the M-CSF and/or GM-CSF added to the medium will be of porcine origin.

For example, the cells may be suspended in a medium that contains M-CSF and/or GM-CSF and a CD16 ligand. Alternatively, it is also possible to add M-CSF/GM-CSF and the CD16 ligand some time after start of cell culturing. The culture medium used in step (b) of the above method can be any medium that has been described in the literature as suitable for use in the culturing of monocytes and/or macrophages. Suitable culturing media include, for example, the PromoCell Macrophage Generation Medium (PromoCell GmbH, Heidelberg, Germany), Dulbecco's modified Eagle's medium (DMEM), DMEM:F12 blend, Medium 199, or RPMI-1640 medium. The culture medium preferably is a chemically defined medium. Apart from M-CSF/GM-CSF, the culture medium can contain other factors to promote the survival and differentiation of Mregs, including: growth factors and cytokines, such as epidermal growth factor (EGF), or IL-4; fatty acids, cholesterol and other lipids; vitamins, transferrin and trace elements; insulin, glucocorticoids, cholecalciferol or ergocalciferol, and other hormones; nonspecific immunoglobulin and other plasma proteins. In a preferred embodiment, the culture medium is RPMI-1640 or a medium derived therefrom.

Usually, the concentration of M-CSF in the culture medium in step (b) of the above method is in the range of 1-100 ng protein per ml medium. Time-course experiments to measure the amount of M□CSF in the culture medium revealed that M□CSF was consumed or degraded over time, such that cultures with an initial dose of 5 ng/ml M□CSF contained sub□physiological concentrations by day 2 of culturing; in contrast, cultures with an initial dose of 25 ng/ml M□CSF maintained concentrations of >10 ng/ml throughout a 7□day culturing period. Thus, the concentration of M-CSF in the culture medium will normally be in the range of 20-75 ng/ml, such as 20-25 ng/ml. Where GM-CSF is used instead of M-CSF, the same concentrations can be used in the medium as outlined above in the context with M-CSF. Since GM-CSF appears to be more potent compared to M-CSF, a concentration of GM-CSF of 0.1-100 ng protein per ml medium is suggested. In cases where both M-CSF and GM-CSF are used in the medium, the overall concentrations of these two growth factors will be in the above-mentioned range, i.e. in the range of 20-75 ng/ml.

Apart from the M-CSF and/or GM-CSF, the culture medium used in step (b) of the above method also comprises a CD16 ligand. Stimulation of the CD16 cell surface receptor on the monocytes is required to induce their differentiation into Mreg cells. Stimulation of the CD16 cell surface receptor can be achieved by the addition of a human or non-human immunoglobulin, and more preferably a human immunoglobulin, or a fragment thereof. The immunoglobulin fragment can be, for example, an Fc fragment of an immunoglobulin. It is assumed that the immunoglobulin acts through FcyRIII (CD16) to induce the Mreg phenotype. A simple way to achieve stimulation of the CD16 ligand is the addition of human serum to the culture medium. Thus, the medium used for generating the Mreg cells may contain 1-20% human AB serum.

Where the Mreg-derived vesicles are intended for use in therapeutic applications in which the induction of angiogenesis is desired, the medium used for culturing the monocytes in step (b) of the above method may comprise, apart from M-CSF/GM-CSF and the CD16 ligand, a toll-like receptor (TLR) ligand, such as lipolysaccharide (LPS), monophosphoryl lipid A (MPLA) or High Mobility Group Box protein 1 (HMGB 1) to enhance the production of angiogenic factors like VEGF-A. The TLR ligand can be added to the culture medium in a concentration range of 1000 ng/ml to 1 µg/ml. The TLR ligand can be added at any stage of the production method. It can be present in the initial medium which is used for culturing the monocytes, i.e. at day 0 of the culture, or it can be added at a later stage, e.g. at day 5, 6 or 7 of the culture. Preferably, the TLR ligand is added simultaneously with the addition of the IFN-γ.

In step (c) of the above method, the cells are contacted with the cytokine interferon gamma (IFN-γ). The choice of the IFN-γ used in the method of the invention will depend on the origin of the monocytes which are subjected to the differentiation process. If human monocytes are differentiated into Mregs, the IFN-γ added will normally be recombinant human IFN-γ. The amount of IFN-γ to be added to the monocyte culture will be in the range of 5-100 ng/ml. An amount of 25 ng IFN-γ per ml culture medium is particularly suitable.

The IFN-γ can be added to the medium simultaneously with the M-CSF/GM-CSF and the CD16 ligand which means that the cytokine may be added, e.g., at the time when the culture of the monocytes is initiated. In such a method, the monocytes to be differentiated will be cultured in the presence of M-CSF/GM-CSF, the CD16 ligand and IFN-γ for the entire culturing period. Normally, however, the culturing period in the presence of IFN-γ is considerably shorter than the culturing period in the presence of M-CSF/GM-CSF, which means that the IFN-γ is normally added after the cells have been cultured for e.g. 3 days in the presence of M-CSF/GM-CSF, and culturing in the presence of IFN-γ is then continued for another 18-72 hours.

In step (d) of the above method, the macrophage-derived extracellular vesicles are obtained from the culture medium. Particularly good results have been achieved when the cells are cultured for 6 days in the presence of M-CSF/GM-CSF and the CD16 ligand, and subsequently pulsed with IFN-γ for 18-24 hours. The macrophage-derived extracellular vesicles, and optionally also the differentiated Mregs, can then be harvested at day 7. For harvesting the vesicles, different methods can be used such as centrifugation or filtration. For example, the culture medium obtained at the end of step (c) of the above method, which contains both the Mreg cells and the vesicles derived from these cells, can be subjected to centrifugation at low speed, e.g. at 400xg or less, 300xg or less, 200xg or less, or 100xg or less for a time ranging from 10-60 minutes.

Preferably, step (d) involves centrifugation at 500xg for 10-20 minutes. The pellet obtained from this centrifugation essentially consists of complete Mreg cells. These can be further processed or stored for further use. The supernatant obtained from this centrifugation essentially consists of extracellular vesicles and soluble components, including L-EV-Mreg and smaller extracellular vesicles. This supernatant can be subjected to centrifugation at a higher speed, e.g. at 3000xg or more, 4000xg or more, or 5000xg or more for a time ranging from 30-180 minutes. Preferably, step (d) involves centrifugation at 4000xg for 20-60 minutes. The pellet obtained from this centrifugation essentially consists of L-EV-Mreg, while smaller vesicles and soluble factors can be found in the supernatant.

The L-EV-Mreg isolated in this way may be washed with a buffer which is generally compatible for use with macrophages, such as Phosphate Buffered Saline (PBS) supplemented with 5% human serum albumin. The vesicles can be transferred and stored in a transfusion bag, a glass infusion device or in another closed-system container which allow for the transportation.

The extracellular vesicles of the invention are derived from immunoregulatory macrophages which can be differentiated from monocytes. It is particularly preferred that the vesicles of the invention have been derived from immunoregulatory macrophage cells that express the markers CD258, DHRS9 and IDO. The combination of these three markers provides a reliable description for immunoregulatory Mregs which separates these cells from other macrophages.

The marker CD258, which is also referred to in the literature as LIGHT or TNFSF14, is a secreted protein of the TNF superfamily. The human sequence of CD258 can be found under NCBI gene_ID 8740. IDO refers to the Indoleamine 2,3-dioxygenase. The sequence of the human gene encoding this marker can be found under NCBI gene_ID 3620. Synonyms for IDO are IDO1 or INDO. DHRS9 is a retinol dehydrogenase of the SDR family of retinol dehydrogenases. The sequence of the human gene encoding this marker can be found under NCBI gene_ID 10170.

In a preferred embodiment of the invention, the immunoregulatory macrophage which gives rise to the extracellular vesicles of the invention further expresses at least one of the markers selected from the group consisting of TGFβ1 and PAEP. TGFβ1 refers to the transforming growth factor beta, a multifunctional cytokine belonging to the transforming growth factor superfamily. The sequence of the human gene encoding this marker can be found under NCBI gene_ID 7040. PAEP refers to the progestagen-associated endometrial protein. The sequence of the human gene encoding this marker can be found under NCBI gene_ID 5047.

In addition, the immunoregulatory macrophage which gives rise to the extracellular vesicles of the invention may express common macrophage markers. Therefore, the Mreg immunoregulatory macrophage from which the extracellular vesicles are derived may further express at least one of the markers selected from the group consisting of CD33, CD11b and HLA-DR.

In a particularly preferred embodiment, the immunoregulatory macrophage which gives rise to the extracellular vesicles of the invention expresses one of the following marker patterns:
(1) CD258, DHRS9, IDO;
(2) CD258, DHRS9, IDO, TGFβ1;
(3) CD258, DHRS9, IDO PAEP;
(4) CD258, DHRS9, IDO, TGFβ1, PAEP;
(5) CD258, DHRS9, IDO, TGFβ1, PAEP, CD33;
(6) CD258, DHRS9, IDO, TGFβ1, PAEP, CD33, CD11b;
(7) CD258, DHRS9, IDO, TGFβ1, PAEP, CD33, CD11b, HLA-DR;
(8) CD258, DHRS9, IDO, CD33, CD11b, HLA-DR;
(9) CD258, DHRS9, IDO, CD33, CD11b, HLA-DR, TGFβ1;
(10) CD258, DHRS9, IDO, CD33, CD11b, HLA-DR, PAEP.

Vesicles derived from an immunoregulatory macrophage that expresses CD258, DHRS9, IDO, TGFβ1 and PAEP are particularly preferred herein.

The expression of markers by the macrophage that produces the vesicles of the invention can be determined on the mRNA or protein level. In a particularly preferred embodiment, detection of markers is made at the transcriptional level. Suitable methods for monitoring gene expression at the transcriptional level include those which allow for the quantitative or semi-quantitative detection of mRNA levels, for example, quantitative RT-PCR (e.g., TaqMan^{™} RT-PCR), real-time RT-PCR, Northern-Blot analysis or other methods which are generally known in the art.

Detection at the transcriptional level usually requires as a first step the isolation of mRNA from the macrophages to be analyzed. Methods for isolating RNA, such as mRNA, are well known in the art and are discussed in detail in the literature (see, for example, Sambrook et al. (1989), Molecular Cloning - A Laboratory Manual, Cold Spring Harbor Laboratory, Cold Spring Harbor, New York and Ausubel et al. (1994), Current Protocols in Molecular Biology, Current Protocols Publishing, New York). Such methods normally involve the lysis of the cells. Cell lysis may be performed by use of detergents which are capable of disrupting the plasma membrane of the cells. For example, buffers containing guanidine thiocyanate and/or SDS may be used for cell lysis. The methods may comprise steps in which the DNA of the cells is enzymatically digested in order to obtain pure RNA without traces of DNA which might interfere with further downstream applications, such as the monitoring of expression levels. Inhibitors of enzymes which lead to the degradation of the RNA may also be added to the lysis buffer. Kits for preparing highly purified RNA are available from several manufacturers, such as Qiagen, Ambion, Stratagene, Clontech, Invitrogen, Promega, and others.

The RNA isolated from the cell or tissue sample by use of commercial kits will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex^{™} matrix can be performed, as mRNAs contain a poly(A) tail at their 3' terminus (see, for example, Ausubel et al. (1994), Current Protocols in Molecular Biology, Current Protocols Publishing, New York).

Commonly used methods for detecting marker expression in immunoregulatory macrophages at the transcriptional level include, for example, RT-PCR, TaqMan RT-PCR, microarray analysis, and the like, as explained in more detail in WO 2019/053091 A1.The primers or probes used in the PCR or RT-PCR detection methods will be designed to allow the specific hybridization to and the subsequent amplification of the target sequence within the respective marker gene, e.g. the CD258, DHRS9, or IDO gene. The skilled person will be readily able, based on the disclosure in WO 2019/053091 A1, to design oligonucleotide primers and/or probes which can be used for detecting the expression of the respective marker genes.

Where high levels of mRNA of the markers, e.g. CD258, DHRS9 and IDO, are detected, it can be assumed that this cell is an Mreg. IDO and DHRS9 are intracellular markers and can be detected, e.g. by RT-PCR. While it is possible that also non-Mreg cells, such as resting macrophages, may express one or more than one of the markers indicated above to some degree, such expression will be comparatively low. According to the invention, it is hence preferred that the expression of the markers by non-Mreg cells, in particular by a resting macrophage, is used as a negative control. Measuring marker expression at an RNA level by quantitative RT-PCR which is at least about 50%, at least about 75%, at least about 150%, at least about 200%, at least about 250%, at least about 300%, at least about 350%, at least about 400%, at least about 450%, at least about 500%, at least about 550%, at least about 600%, at least about 750%, or at least about 1000% higher than the respective amounts in the negative control, i.e. in the resting macrophages, clearly indicates that the tested cell is an Mreg cell.

In yet another particularly preferred embodiment, detection of the Mreg markers, e.g. CD258, DHRS9 and IDO, involves the detection of marker gene expression at the translational level, i.e. on the protein level. This means that the amount of protein of the respective marker, such as CD258, DHRS9 and IDO, is determined. The amount of marker protein may be determined by any suitable method which is able to specifically detect proteins in a biological sample. The detection of the protein may be based on a molecule that binds specifically to the protein or it may be based on the separation of the protein from other proteins that are present in the sample. Molecules that specifically bind to marker proteins include antibodies and antibody fragments with binding activity for the respective markers. Numerous antibodies can be commercially obtained which are directed to marker proteins like CD258, DHRS9 and IDO. Such antibodies or fragments thereof may be used for detection of the marker proteins in a Western blot, quantitative Western blot, enzyme-linked immunosorbent-assay (ELISA), polarization analysis, (quantitative) surface plasmon resonance (SPR), or in immunohistochemical methods, including quantitative electron microscopic methods. In a particularly preferred embodiment of the invention, the detection of the markers involves an ELISA. Other methods which are able to detect specific binding include, for example, fluorescence resonance energy transfer (FRET). Methods which allow the quantitative detection of the marker proteins by separating the protein from other components in the biological sample include quantitative mass spectrometric methods, electrophoretic methods, such as two-dimensional gel electrophoresis, and chromatographic methods, such as size-exclusion chromatography or ion-exchange chromatography.

As stated above, if the markers are to be detected on the protein level, it is particularly preferred that such detection involves flow cytometry. Flow cytometry may involve measuring fluorescence intensity produced by fluorescent-labeled antibodies that detect markers on the cell surface. Although it can also be used for detecting intracellular markers, such detection is less desirable, since the antibody must permeate the cell which normally kills the cell.

As stated above, it is possible that also non-Mreg cells may produce the Mreg markers mentioned above, e.g. CD258, DHRS9 and IDO, in low amounts. Accordingly, protein levels of non-Mreg cells, such as a non-regulatory macrophage, should also be measured to provide a negative control. If the protein levels for tested cells reveal that marker expression is higher than the negative control, this allows the conclusion that the tested cell is a Mreg. Specifically, measuring marker expression at a protein level is at least about 50%, at least about 75%, at least about 150%, at least about 200%, at least about 250%, at least about 300%, at least about 350%, at least about 400%, at least about 450%, at least about 500%, at least about 550%, at least about 600%, at least about 750%, or at least about 1000% higher than the respective amounts in the negative control, i.e. in the non-Mreg cell, such as a non-regulatory macrophage, clearly indicates that the tested cell is an Mreg cell. Measuring marker expression at protein level is preferably performed by quantitative ELISA.

In a fourth aspect, the invention refers to the use of the vesicles according to the first aspect of the invention or the pharmaceutical composition according to the second aspect of the invention in medicine, i.e. for therapeutic purposes. It is demonstrated herein that the macrophage-derived extracellular vesicles exhibit essentially the same characteristics as the Mreg cells from which they are derived. Hence, the vesicles exert a number of therapeutically useful properties, such as immunosuppressive, immunoregulatory, angiogenic and anti-inflammatory properties, which make them highly suitable for being used in immunosuppressive, anti-inflammatory or tissue-reparative therapies. For example, like the macrophages from which they are derived, extracellular vesicles of the invention are thought to be T cell-suppressive and mediate an active deletion of activated T cells. As such, the vesicles are highly suitable for use as an adjunct immunosuppressive therapy in a variety of immunologically □ mediated diseases and in the context with transplantation of organs or tissues.

Accordingly, in one embodiment of the invention, the extracellular vesicles according to the first aspect of the invention or a pharmaceutical composition according to the second aspect of the invention is used in a method of suppressing transplant rejection and/or prolonging transplant survival in a subject receiving a transplant. The invention thus also refers to a method of suppressing transplant rejection and/or prolonging transplant survival in a subject receiving a transplant, comprising (i) the administration of an effective amount of the macrophage-derived extracellular vesicles according to the first aspect of the invention, or (ii) the administration of a pharmaceutical composition according to the second aspect of the invention. Preferably, the transplant is an organ, tissue or cell transplant. The type of organ to be transplanted is not limited according to the invention, but it will preferably be a kidney, liver, heart, lung, or pancreas. It is particularly preferred that the organ to be transplanted to the recipient is a human organ.

If the transplant is a tissue transplant rather than an organ transplant, the tissue to be transplanted into the recipient is not particularly limited. The rejection of any tissue that was derived from an allogeneic donor in the recipient can be prevented or ameliorated by the macrophage-derived extracellular vesicles of the invention. The tissue to be transplanted will preferably be a human tissue, such as an intestinal, cornea, skin, composite tissue, bone marrow, or pancreatic islet tissue.

The macrophage-derived vesicles of the invention can also support the transplantation of cells into a recipient by suppression of the immune response in the recipient. Where the transplant is a cell transplant, the nature of the cell to be transplanted is generally not limited, but it is preferred that the cell to be transplanted is selected from the group consisting of an adult stem cell transplant, an isolated hepatocyte transplant or a leukocyte cell transplant. The macrophage-derived vesicles of the invention may also contain soluble factors that promote the homing and engraftment of adult stem cells, such as cathelicidin. In a preferred embodiment of this invention, the vesicles are used to facilitate engraftment of haematopoietic stem cells (HSC) after bone marrow or HSC transplantation.

To suppress transplant rejection in the recipient and induce acceptance of an allogeneic organ, tissue or cell transplant, the vesicles of the invention or a pharmaceutical composition comprising said vesicles can be administered intravenously by injection or infusion, as described above. The injection or infusion can be given either pre-operatively or post-operatively. If the vesicles are administered pre-operatively, they will be administered to the recipient at least one time, preferably two times, and more preferably three times before the operation. It is preferred that the vesicles are administered to the recipient not earlier than one week before operation, e.g. 6 days, 5 days, 4 days, 3 days, 2 days, or 1 day before operation. If the vesicles are administered post-operatively, a first administration will be given preferably within 24 hours after operation, more preferably within 36 hours, 48 hours, 60 hours, or 72 hours after operation. In stably immunosuppressed transplant recipients, vesicle therapy may be administered at any time after transplantation. Alternatively, vesicles may be administered to transplant recipients undergoing acute or chronic transplant rejection. The vesicles are then capable of suppressing the T-cell response of the recipient's immune system against the transplant and to persist in the recipient's body for a sufficiently long period of time to confer long-term transplant acceptance to the recipient.

When using the vesicles of the invention for suppressing transplant rejection or prolonging transplant survival in a subject receiving a transplant, the transplant will normally be an allogeneic transplant, i.e. a transplant which originates from a donor that, although genetically different, belongs to the same species as the recipient. In this case, the vesicles are derived from macrophages which had been generated based on blood monocytes that were obtained from said donor. The monocytes can be obtained from a living donor or a dead donor. In case of a dead donor, i.e. a body donation, the body of the donor is normally flushed with a perfusion medium by canalisation of the principal artery for the purpose of organ preservation. The venous blood is removed from the body and can be collected for preparing macrophage-derived extracellular vesicles as described herein. If extracellular vesicles are used that were prepared based on monocytes that had been obtained from a deceased donor, a rejection of the transplanted organ can be prevented by the administration of immunosuppressants which are routinely used for this purpose during organ transplantation.

In another embodiment, the macrophage-derived extracellular vesicles according to the first aspect of the invention or a pharmaceutical composition according to the second aspect of the invention are used in a method of promoting or sustaining the engraftment or effect of regulatory T cell cell-based medicinal products. The invention thus also refers to a method of promoting or sustaining the engraftment or effect of regulatory T cell cell-based medicinal products in a subject comprising (i) the administration of an effective amount of macrophage-derived extracellular vesicles according to the first aspect of the invention, or (ii) the administration of a pharmaceutical composition according to the second aspect of the invention.

Apart from the immunoregulatory and immunosuppressive properties, the macrophage-derived vesicles of the invention have anti-inflammatory properties which render them highly useful for the amelioration of inflammatory processes. Accordingly, the vesicles provided herein are also useful for treating diseases or disorders that are characterized by a deregulated immune status or an excessive inflammation reaction. Such diseases or disorders include, for example, autoimmune diseases, inflammatory diseases and hypersensitivity reactions.

Thus, in yet another embodiment of the invention, the vesicles according to the first aspect of the invention or the pharmaceutical composition according to the second aspect of the invention are used in a method of treating or preventing an autoimmune disease, an inflammatory disease, or a hypersensitivity reaction.

Where the macrophage-derived extracellular vesicles are used for treating an autoimmune disease, said disease may be (a) principally T cell-mediated, (b) principally antibody-mediated or (c) principally mediated by other cellular components of the immune system. The disease may be a local or systemic autoimmune condition. The type of autoimmune conditions to be treated with the macrophage-derived vesicles is not limited and includes systemic lupus erythematosus (SLE), scleroderma, Sjögren's syndrome, polymyositis, dermatomyositis, and other systemic autoimmune conditions; rheumatoid arthritis (RA), juvenile rheumatoid arthritis, and other inflammatory arthritides; ulcerative colitis, Crohn's disease, and other inflammatory bowel diseases; autoimmune hepatitis, primary biliary cirrhosis, and other autoimmune liver diseases; cutaneous small-vessel vasculitis, granulomatosis with polyangiitis, eosinophilic granulomatosis with polyangiitis, Behcet's disease, thromboangiitis obliterans, Kawasaki disease, and other large-, medium- or small-vessel vasculitides of autoimmune aetiology; Multiple sclerosis (MS) and neuro-immunological disorders; Type I diabetes, autoimmune thyroid dysfunction, autoimmune pituitary dysfunction, and other autoimmune endocrinological disorders; haemolytic anemia, thrombocytopaenic purpura and other autoimmune disorders of the blood and bone marrow; psoriasis, pemphigus vulgaris, pemphigoid and other autoimmune dermatological conditions.

The macrophage-derived vesicles are also effective for treating acute or chronic inflammatory diseases, and diseases with a pathophysiologically significant inflammatory component. The inflammatory disease to be treated may be local or systemic. The type of inflammatory diseases to be treated with the vesicles of the invention is not limited and includes, but is not limited to, arterial occlusive diseases, such as peripheral artery occlusive disease (pAOD), critical limb ischaemia, arteriosclerosis, cerebral infarction, myocardial infarction, renal infarction, intestinal infarction, angina pectoris, and other conditions caused by arterial occlusion or constriction; microvascular angina, also known as cardiac syndrome X; inflammation associated systemic with metabolic disorders, including Type II diabetes and obesity-related metabolic syndrome; dermatological diseases, including eczema. Preferably, the inflammatory disease to be treated is characterized by chronic inflammation of the intima of an arterial wall, e.g. myocardial infarction, stroke, critical limb ischemia vasculitis and pAOD.

The treatment of pAOD is particularly preferred. It is known that pAOD in patients who are unfit for revascularisation procedures, owing either to the extent or location of their arterial occlusions, or to significant co-morbidities, is a severely debilitating and prevalent condition for which amputation is the only therapeutic option. Amputation remains a treatment of last resort and is associated with relatively high mortality, and only a minority of patients subsequently recovers full mobility. It was found earlier that Mreg cells have angiogenic properties. The cells actively promote neovascularisation, i.e. the formation of new blood vessels, through the basal and stimulated expression of pro-angiogenic growth factors, such as VEGF, FIGF (VEGF-D), PDGFB and MDK. In particular, Mreg cells produce high levels of Vascular Endothelial Growth Factor (VEGF) upon stimulation with TLR4 ligands. It was described that Mreg cells induce the expression of the vascular endothelial growth factor C (VEGF-C), an angiogenic factor that effectively stimulates neovascularisation in vivo [27]. The present invention demonstrates that extracellular vesicles derived from Mregs share the angiogenic properties of the Mreg cells from which they are derived. Therefore, these vesicles also promote neovascularisation.

In one embodiment, macrophage-derived extracellular vesicles are injected intramuscularly or subcutaneously into an ischaemic limb. In the ischaemic tissue, the vesicles will inevitably be exposed to microbial components and necrotic tissue components (e.g. HMGB1) that act as TLR4 agonists. Hence, the vesicles can be used to promote tissue regeneration through local secretion of pro-angiogenic growth factors. In another embodiment, the macrophage-derived extracellular vesicles may be stimulated ex vivo with TLR ligands during the manufacturing process to ensure their high-level production of VEGF. Said TLR ligands may include, but are not limited to, lipopolysaccharide (LPS) or monophosphoryl lipid A (MPLA). The pAOD to be treated with the vesicles of the invention may be a pAOD of any grade or category. For example, the pAOD may be a pAOD of grade I, categories 1-4, or grade II-IV pAOD.

The vesicles of the invention are also useful for treating or preventing hypersensitivity reactions. Preferably, the hypersensitivity reaction to be treated or prevented by the macrophage-derived extracellular vesicles of the invention is selected from the group of asthma, eczema, allergic rhinitis, angioedema, drug hypersensitivity, and mastocytosis.

As the extracellular vesicles of the invention have angiogenic properties, their use in other diseases or conditions that require neovascularisation is also contemplated herein. Hence, the invention also relates to vesicles according to the first aspect of the invention or the pharmaceutical composition according to the second aspect of the invention which is used in a method of inducing angiogenesis or vasculogenesis in hypoxic tissues, promoting tissue-repair processes by participating in tissue remodelling, tissue regeneration, preventing or reducing fibrosis, reducing ischemic pain or avoiding major limb amputation. The invention thus also refers to a method of inducing angiogenesis or vasculogenesis in hypoxic tissues, promoting tissue-repair processes by participating in tissue remodelling, tissue regeneration, preventing or reducing fibrosis, reducing ischemic pain or avoiding major limb amputation comprising (i) the administration of an effective amount of macrophage-derived extracellular vesicles according to the first aspect of the invention, or (ii) the administration of a pharmaceutical composition according to the second aspect of the invention.

The extracellular vesicles of the invention are also useful for treating micro- or macroangiopathies in a subject. Hence, the invention also relates to vesicles according to the first aspect of the invention or the pharmaceutical composition according to the second aspect of the invention for use in a method of treating micro- or macroangiopathies of the lower limbs in a subject. The invention also refers to a method of treating micro- or macroangiopathies of the lower limbs in a subject, said method comprising (i) the administration of an effective amount of macrophage-derived extracellular vesicles according to the first aspect of the invention, or (ii) the administration of a pharmaceutical composition according to the second aspect of the invention.

As used herein, a microangiopathy is a blood vessel disease which affects small blood vessels and capillaries in the body, such as small cerebral vessels, small coronary vessels or small vessels in the legs. During microangiopathy, the capillary basement membrane becomes thick and hard which causes obstruction or rupture of capillaries or small arteries which in turn results in tissue necrosis and loss of function. In contrast, a macroangiopathy is a blood vessel disease that affects large blood vessels, such as the arteries. Obstruction of the larger arteries leads to high incidence of heart attacks, strokes and peripheral vascular disease in diabetics. Obstruction of the arteries in the leg often results in ulcers on the feet and legs that slowly heal.

One cause of micro- and macroangiopathies is long-term diabetes mellitus. In diabetic patients, high blood glucose levels cause the endothelial cells of the blood vessels to absorb more glucose than normal. These cells then form more glycoproteins on their surface than normal, and also cause the basement membrane in the vessel wall to grow abnormally thicker and weaker. As a result, the vessel wall become leaky and reduces the flow of blood through the body such that some tissues do not receive sufficient oxygen and are damaged.

According to the invention, the microangiopathy to be treated is preferably selected from the group of diseases consisting of angiitis, arteritis, angiodysplasia, atrophy blanche, dermatosclerosis, Determann syndrome, diabetic angiopathy, endangiitis obliterans, erythromelalgia, fibro muscular dysplasia, malum perforans, Mönckeberg Sclerosis, Osler's disease, compartment syndrome, Paget-von-Schroetter syndrome, Raynaud's syndrome, and leg ulcers. In a particularly preferred aspect, the microangiopathies to be treated according to the invention are leg ulcers. As used herein, leg ulcers include diabetic leg ulcers and venous leg ulcers.

According to the invention, the macroangiopathy to be treated with the extracellular vesicles of the invention is preferably selected from the group of aneurysm, dissection, atherosclerosis, atherothrombosis, peripheral arterial occlusive disease (pAOD), claudicatio intermittens, necrosis and gangrene, vascular malformation, Leriche syndrome, or compression syndrome.

In another aspect, the extracellular vesicles of the invention are for use in a method of promoting surgical, traumatic or other wound healing in a subject. Vesicle therapy may be used to promote healing of acute or chronic wounds, optionally in conjunction with conventional management (i.e. cleaning, closure and dressing). The wounds to be treated may be open or closed wounds. Open wounds may include incisions, lacerations, abrasions, avulsions, penetrating injuries or punctures. Closed wounds may include crush injuries or haematomas. Incisions may be traumatic or iatrogenic (i.e. surgical incisions). The extracellular vesicles of the invention may be used to promote engraftment of autologous or allogeneic skin grafts. The extracellular vesicles of the invention may be used, either in conjunction with conventional management or not, for the healing of burns that are caused by exposure of skin to heat, extreme cold, chemicals, friction, radiation or electrical current. The statements made herein in connection with the treatment of ulcers likewise apply to the treatment of wounds or burns.

In yet another aspect of the invention, the macrophage-derived extracellular vesicles of the invention can be used for producing immunoregulatory T cells. It has been described earlier that T cells that had been co-cultured with Mregs develop a regulatory T cell phenotype that suppresses the proliferation of effector T cells and inhibit the maturation of dendritic cells. Accordingly, the macrophage-derived extracellular vesicles of the present invention can also be used instead of Mregs for the production of immunoregulatory T cells.

Thus, according to a fifth aspect, the invention refers to a process for preparing an immunoregulatory T cell, said process comprising:
(a) obtaining T cells of a subject;
(b) co-culturing the T cells with macrophage-derived extracellular vesicles as described herein above;
(c) obtaining the immunoregulatory T cells from the culture medium.

The immunoregulatory T cells obtained by the above method can be used, either alone or in combination with Mregs or the macrophage-derived extracellular vesicles of the invention, for the treatment of any of the diseases or disorders discussed elsewhere herein. In a first step of the above process, T cells from a blood sample of a subject are obtained. The cells can be obtained, e.g. from a blood sample or apheresate, or from the subject's tissue, e.g. from bone marrow or the spleen. The cells can be obtained by conventional methods, e.g. in case of cells from the blood by venepuncture. The T cells used in the above method will be, for example, CD3+ T cells or subsets thereof. Before being co-cultured with the macrophage-derived extracellular vesicles, the CD3+ T cells can be purified or enriched by conventional methods, e.g. by magnetic microbead separation or flow cytometry sorting. Also, the T cells can be activated prior to the co-culturing step. For example, the T cells can be treated with CD3/CD28 activation beads, i.e. superparamagnetic beads which are similar in size to antigen-presenting cells and are covalently coupled to anti-CD3 and anti-CD28 antibodies, for this purpose.

The T cells are then contacted with macrophage-derived vesicles of the present invention. The cells can be contacted in different vesicle:Treg ratios. For example, the cell fractions can be contacted in a vesicle:Treg ratio of between 1:1 to 50:1, preferably between 5:1 and 10:1. More preferably, vesicle:Treg ratio is about 15:1. Different media can be used for the co-culturing method. The media can be those described above in connection with the method for preparing Mreg cells. In a preferred embodiment the medium is RPMI1640 from Gibco which is supplemented with 10% human serum. The medium may contain further additives such as M-CSF and/or GM-CSF, preferably human recombinant M-CSF and/or GM-CSF. The amount of M-CSF and/or GM-CSF will be in the range mentioned elsewhere herein, e.g. 5-100 ng/ml, preferably 20-25 ng/ml. The medium may also contain other additives, such as Glutamax in an amount of 1-5 mM, preferably 2 mM.

The T cells will be co-cultured with the macrophage-derived vesicles for 1-8 days, preferably for at least 3 days, at least 4 days, or at least 5 days, and more preferably for at least 3 days. After the pre-determined culturing period, the immunoregulatory T cells are obtained from the culture medium. For example, the T cells can be isolated by conventional methods, e.g. by enriching CD4+ CD25+ TIGIT+ FoxP3+ Tregs. The activation status of the T cells can be determined, e.g. by FACS. If necessary, the cells can be further formulated to pharmaceutical products.

### BRIEF DESCRIPTION OF THE FIGURES

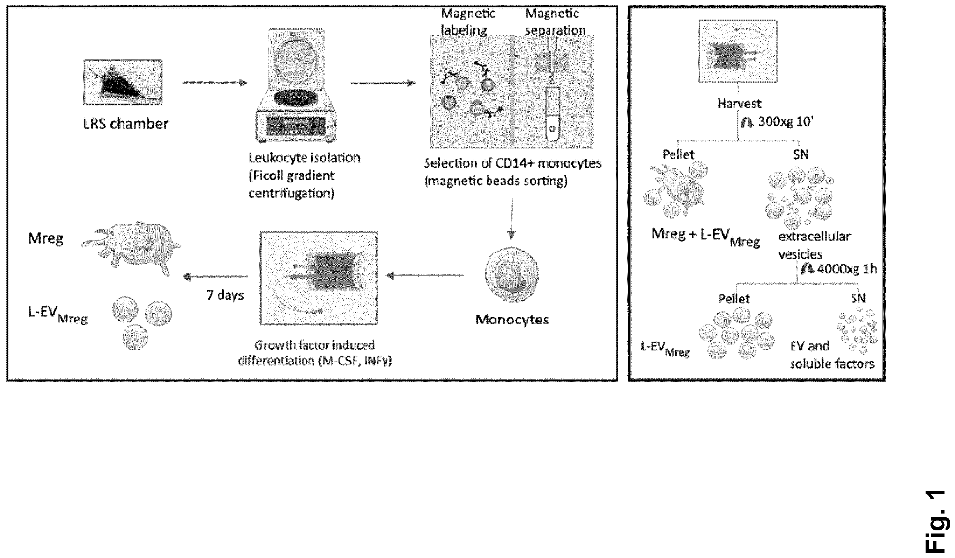

Figure 1 shows a schematic representation of in-vitro differentiation of human monocytes to Mreg and L-EV-Mreg (left) as well as differential centrifugation steps leading to L-EV-Mreg enriched pellets (right). SN, supernatant; EV, extracellular vesicles.

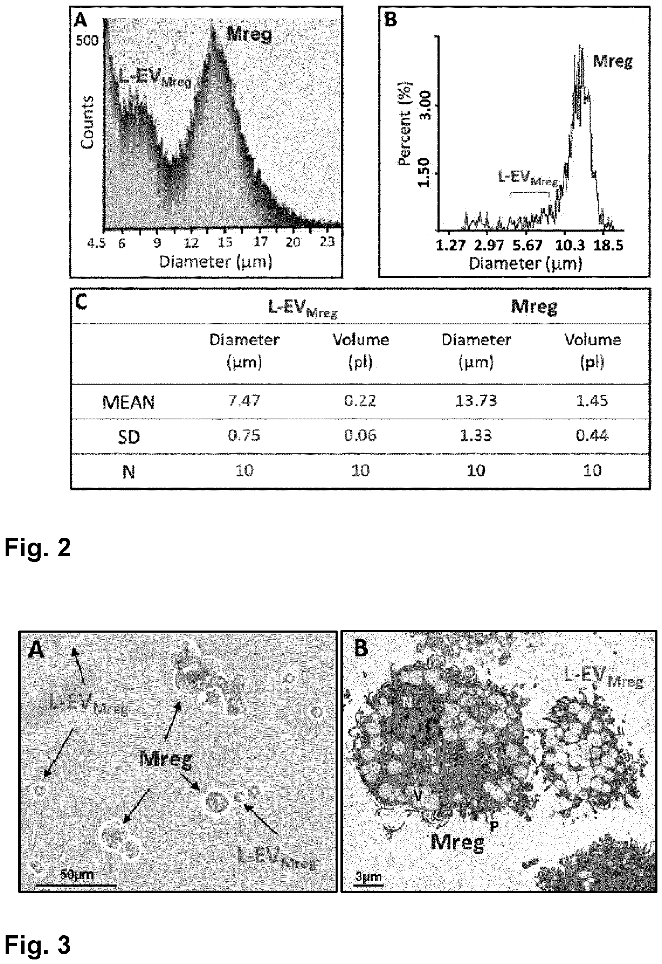

Figure 2 shows the results of the vesicle and cell analysis. A. Representative analysis of a sample containing Mreg and L-EV-Mreg using the MOXI counter. B. Representative analysis of a sample containing Mreg and L-EV-Mreg using the Nucleocounter. Nucleus-lacking vesicles are not detected using the Nucleocounter method. C. Quantitative results from vesicle and cell analysis.
Figure 3 shows the morphology of L-EV-Mreg observed by microscopy. A. Brighfield microscopy after harvest on day 7. B. Transmission electron microscopy after harvest on day 7. L-EVMreg share several morphological features of Mreg cells such as numerous vesicles and/or vacuoles as well as pseudopodia-like extensions. N, nucleus; V, vesicle/vacuole; P, pseudopodia.

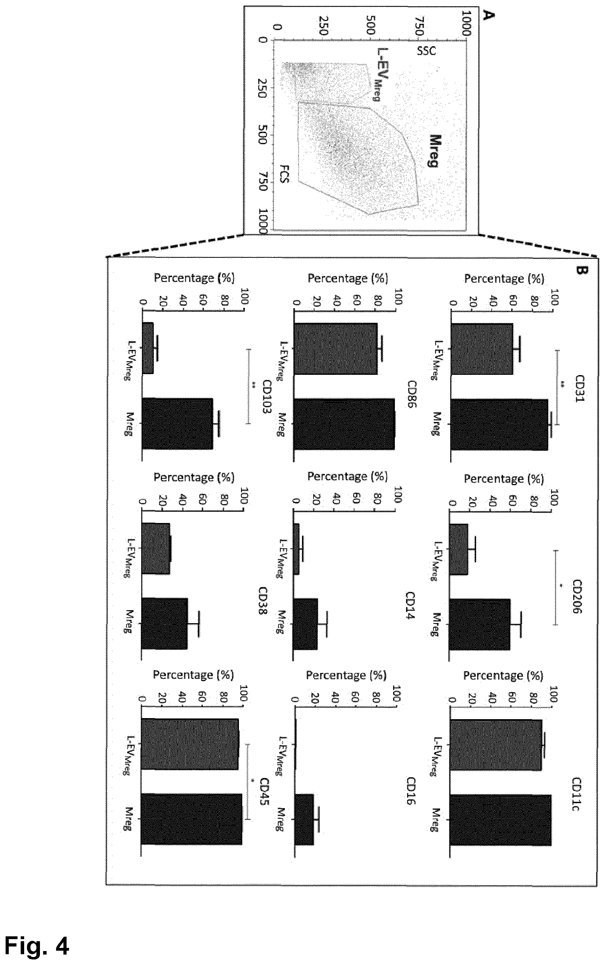

Figure 4 shows the results of the cell surface marker characterization of L-EV-Mreg. A. Representative scatter plot (FCS vs. SSC) showing L-EV-Mreg and Mreg populations at day 7. B. Flow cytometry analysis of surface marker characteristics of L-EV-Mreg and Mreg. FSC, forward scatter; SSC, sideward scatter; *, P<0.05; **, P<0.01.

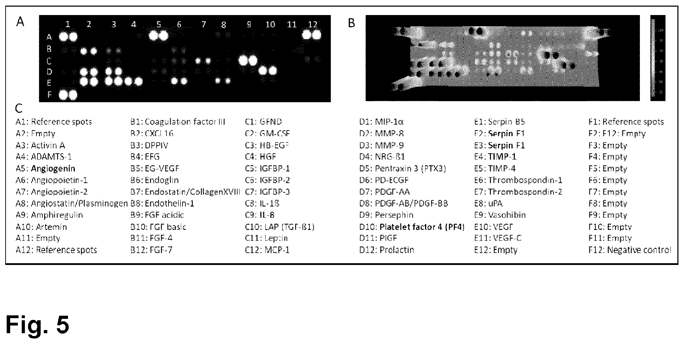

Figure 5 shows the results of the characterization of angiogenic proteins in L-EV-Mreg. A. Representative proteome profiling array membrane incubated with sonicated L-EV-Mreg showing the relative abundance of 55 proteins involved in angiogenesis. Each protein is represented by duplicate spots on the respective membrane. B. Three-dimensional heatmap analysis of A. C. Arrangement of protein spots on the membrane. Proteins with the highest signal intensities are depicted in bold.

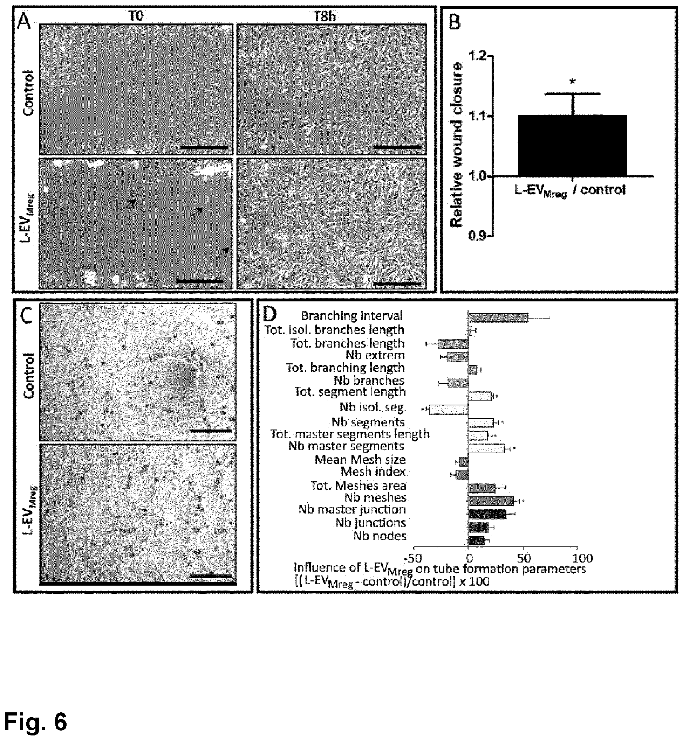

Figure 6 shows the effects of L-EV-Mreg on in-vitro wound healing and angiogenesis. A. Photomicrographs of a representative wound healing assay employing human endothelial cells (HUVEC) with addition of 10⁶ L-EV-Mreg /ml and without addition of L-EV-Mreg. Note the presence of numerous L-EV in the treatment group (arrows). Scale bars denote 250µm. B. Statistical analysis of the effect of L-EV-Mreg addition on relative wound closure (control =1) after 8h. The column denotes the mean ± SD of 3 independent experiments; *, P<0.05 (one sample student t-test vs. 1). C. Representative images of tube formation analysis employing HUVEC cell cultures with and without the addition of L-EV-Mreg. Scale bars denote 500µm. D. Influence of L-EV-Mreg addition on various tube formation parameters. Horizontal columns denote the mean ± SD of 3 independent experiments. *, P<0.05; **, P<0.01 (one sample student t-test vs. 0). Green color, branches related parameters; yellow color, segments related parameters; cyan color, meshes related parameters; blue color, nodes related parameters; red color, junction related parameters.

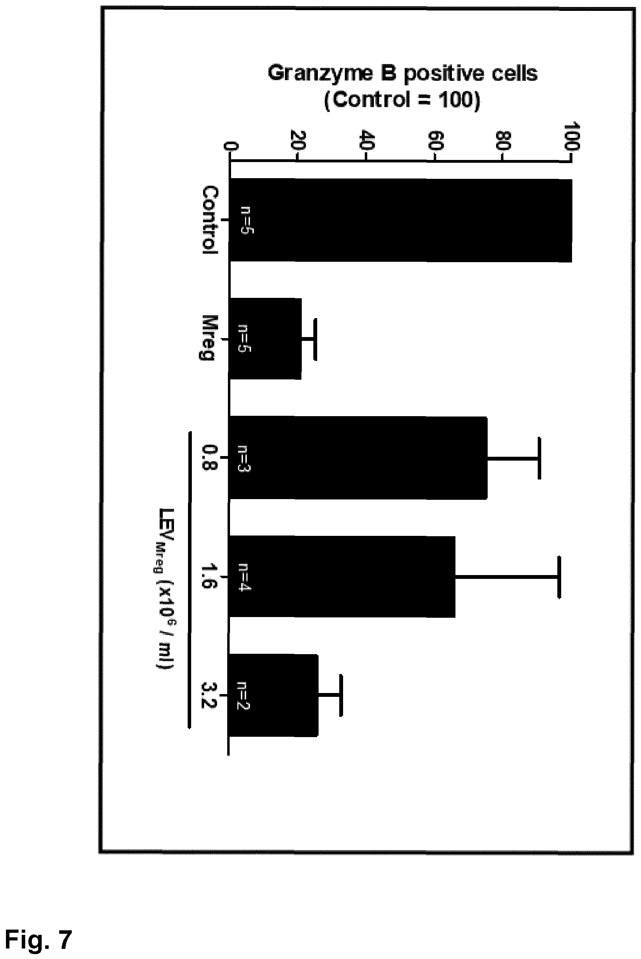

Figure 7 shows the results of the T cell expansion inhibition experiments. T cells were activated by CD3/CD28 beads and subsequently incubated with Mreg cells or different concentrations of L-EV-Mreg. The addition of Mreg or L-EV-Mreg led to a significant reduction in the amount of granzyme B positive cells.

### EXAMPLES

All experiments were approved by the local Ethics Committee of the University Medical Center Schleswig-Holstein, Kiel, Germany (protocol identification: D519/18 and D518/13). The Mregs were manufactured in accordance with current GMP principles for the production of sterile medicinal products. Attention is paid at every processing step that products, materials and equipment are protected against contamination and impurities.

All experiments were carried out with L-EVMreg derived from 3-9 Mreg preparations from different donors. The statistics software GraphPad Prism 5.01 for windows (GraphPad Software: San Diego, USA) was used to compare groups. All data were tested for normality using the Kolmogorov-Smirnov test. In cases normality was not obtained, the data were transformed (arcsin of square root of x) and analyzed using one-way ANOVA with Tukey-test. A P-value <0.05 was considered significant. All values are expressed as mean ± standard deviation (SD) or ± standard error mean (SEM) as specified in each case.

### Example 1: Mreg differentiation and isolation of large extracellular vesicles

Peripheral blood mononuclear cells (PBMC) were obtained from leukocyte reduction system (LRS) chambers provided by the Department of Transfusion Medicine (University Hospital of Schleswig-Holstein, Kiel, Germany). Monocytes were isolated and differentiated to Mreg as shown in Figure 1 (left panel). Briefly, PBMC were purified through a Ficoll-Paque PLUS gradient (GE Healthcare, Chicago, USA) and monocytes were recovered using a CD14 positive magnetic bead cell sorting system (Miltenyi, Bergisch Gladbach, Germany) following the manufacturer's protocol. The isolated CD14+ monocytes were cultivated in bags (Miltenyi) at 0.83 × 10⁶ cells/ml with RPMI 1640 medium containing GlutaMax (GIBCO, Billings, MT, USA) supplemented with 10% human AB-Serum (Access Biological, Vista, CA, USA) and 4200 IU/ml human M-CSF (R&D Systems, Wiesbaden, Germany).

The culture bags were placed in an incubator under standard culture conditions (humidified atmosphere with 5% carbon dioxide/95% air, at 37°C). After 6 days in culture, 500 IU/ml of human interferon (IFN) γ (R&D Systems, McKinley Place MN, USA) was added to the cultures and cells were incubated for additional 24h. On day 0 (after cell seeding) and day 6 (after addition of IFNγ) cell cultivation bags were inverted ("flipped"). On day 7 Mreg were harvested and separated from culture medium by centrifugation at 300 x g for 10 min at room temperature (Figure 1, right panel).

The pellets containing Mreg were resuspended in PBS and subjected to further analyses. The remaining culture supernatant containing EV was further centrifugated at 4000 x g for 1h at 4°C and the L-EV-Mreg containing pellet was resuspended in PBS and subjected to further analyses. Alternatively, L-EV-Mreg were resuspended 1:1 in Cryostore 5 cryopreservation medium (Stemcell technologies, Cologne, Germany) and stored at -80°C until further use.

Results: It was found that L-EV can be detected in Mreg cultures at the end of the differentiation period on day 7. The average yield of L-EV was 1.97±0.64 L-EV per Mreg cell (L-EVMreg/Mreg). L-EVMreg/Mreg was negatively correlated with Mreg recovery (ratio of number of harvested Mreg at day 7 and number of CD14+ monocytes seeded at day 0) and lactate concentration of the culture medium, positively correlated with the pH of the culture medium and not correlated to the glucose concentration of the culture medium at day 7 (data not shown).

### Example 2: Cell counter and Nucleocounter analysis

For automated cell and vesicle analysis, basic parameters such as the number of particles (cells or vesicles) were evaluated using: (i) A MOXI cell counter (Orflo, Ketchum, ID, USA) which analyses membrane surrounded vesicles and cells within a size between 3 to 20µm based on the Coulter principle; and (ii) a Nucleocounter (NC-200 Chemometec, Allerod, Denmark) which stains the cell's nucleus using 2 different dyes enabling the discrimination between live and dead cells.

Results: While Mreg reveal an average size of 13.46±1.17 µm and an average volume of 1.31±0.34 pl the corresponding L-EVMreg population represent definable vesicles with an average size of 7.34±0.71 µm and an average volume of 0.21±0.06 pl (Figure 2). Note that while L-EV-Mreg are clearly detectable by methods using the Coulter counter principle (MOXI analysis; Figure 2A), they cannot be visualized by nucleic acid staining based analysis (Nucleocounter analysis; Figure 2B), suggesting that Mreg derived L-EV lack double-stranded nucleic acids (i.e. nucleus).

### Example 3: Microscopic analysis

Mreg cells and vesicles were analyzed by brightfield and transmission electron microscopy. For transmission electron microscopy, Mreg and L-EV-Mreg pellets were fixed in 3% glutaraldehyde in PBS for 30 min. After postfixation in 2% osmiumoxide for 2 × 15 min and dehydration in increasing series of ethanol, Mreg and L-EV-Mreg were embedded in araldite overnight. The araldite block was trimmed for ultra-thin sectioning and ultra-thin sections (40-50 nm) were cut using the Ultramicrotome Leica UC7 with a diamond knife (Diatom, Hatfield, PA, USA). Sections were contrasted with uranyl acetate for 15 min as well as lead citrate for 7 min. Analysis was carried out with a transmission electron microscope (Jeol JEM1400plus) coupled to a digital imaging system (Firma TVIPS TemCam-F416).

Results: L-EVMreg can easily be observed by brightfield microscopy in Mreg cultures directly after cell harvest (Figure 3A). Employing high magnification TEM Mreg show the typical appearance of nucleated, pseudopodia containing macrophages with numerous of intracellular vesicles/vacuoles. L-EVMreg are smaller in size, lack nuclei and reveal a defined and homogeneous intravesicular structure consisting of numerous of vesicles/vacuoles in the size range around 1µm (Figure 3B). It should be noted that the cell culture medium containing 10% FCS does not contain any L-EV (data not shown) and that these are generated de novo during Mreg differentiation.

### Example 4: FACS analysis

Fluorescent activated cell sorting (FACS) was performed using the MACS Q10^{™} cytometer (Miltenyi). Specific antibodies and their corresponding isotypes (all from BD Biosciences) were directly conjugated with fluorescein isothiocyanate (FITC): CD31, CD16, CD45, anti-mouse IgG1κ. Conjugated with phycoerythrin (PE): CD86, CD38, CD11c, anti-mouse IgG1κ.

Conjugated with allophycocyanin (APC): CD206, CD103, anti-mouse IgG1κ, CD14, anti-mouse IgG2a. The gating strategy consisted of (i) identification of the main Mreg and L-EV populations based on their size and granularity (FSC/SSC profiles), (ii) exclusion of non-viable cells (by 7-AAD exclusion, BD Biosciences), (iii) identification of Mreg using the respective identity markers and (iv) analysis of the same markers on L-EV.

Results: Flow cytometry experiments were performed using 9 different antibodies against specific cluster of differentiation (CD) molecules, that were also used previously for characterization of Mreg [15]. The presence of these specific markers was analyzed on L-EV-Mreg as well as on Mreg, and both populations showed a similar CD expression pattern for several of the investigated molecules on their surface (CD11c on L-EV-Mreg: 90.33±2.74% and on Mreg: 99.14±0.29%; CD86 on L-EV-Mreg: 81.74±5.11% and on Mreg: 98.54±0.80%; CD14 on L-EV-Mreg: 5.34±4.13% and on Mreg: 24.06±9.22%; CD16 on L-EV-Mreg: 0.70±0.20% and on Mreg: 17.88±5.88%, and CD38 on L-EV-Mreg: 26.93±1.57% and on Mreg 44.56±11.80%). However, compared to Mreg, significantly fewer L-EV-Mreg were positive for CD31 (L-EV-Mreg: 60.62±7.01%; Mreg: 95.50±3.29%; P<0.01), CD206 (L-EV-Mreg: 18.26±7.69%; Mreg: 59.66±10.78%; P<0.05), CD103 (L-EV-Mreg: 10.35±4.27%; Mreg: 68.92±6.92%; P<0.01) and CD45 (L-EV-Mreg: 94.84±0.86%; Mreg: 99.02±0.46%; P<0.05) (Figure 4).

Flow cytometry analyses demonstrated the presence of typical extracellular vesicular membrane markers on the L-EVMreg (LAMP-1, CD9, CD63 and CD81) based on the guidelines for the Minimal Information for Studies of Extracellular Vesicles (MISEV 2018) [24, 25, 26]).

### Example 5: Human angiogenesis proteome profiling array

L-EV-Mreg were semi-quantitatively evaluated for the presence of 55 angiogenesis-related proteins using a human proteome profiler array kit (R&D Systems, Minneapolis, MN, USA) as described in the manufacturer's protocol. Briefly, isolated L-EV-Mreg samples resuspended in PBS were sonicated for 20 min on ice to release intravesicular proteins and were applied to the array membranes carrying antibodies against the respective pro-angiogenic proteins. After incubation, a cocktail of biotinylated antibodies and HRP-streptavidin was added to the membranes and the signals were visualized by chemiluminescence detection, referring to the manual provided. Photographs of the membranes were taken using the Fusion FX Vilber (Vilber Lourmat, Eberhardzell, Germany) and signal intensities were analyzed employing the ImageJ 1.41 software (NIH), and Vilber Smart imaging (Vilber Lourmat).

Results: Proteome profiling was performed to evaluate whether L-EV-Mreg contain intravesicular pro-angiogenic proteins. Semi-quantitative analysis revealed the existence of abundant amounts of potentially pro-angiogenic mediators within L-EV-Mreg. The results are depicted in Figure 5. The evaluation of 55 angiogenesis-related proteins showed detectable levels of 23 proteins (signal intensity > 10% of reference spots). The most abundant proteins detected in L-EV-Mreg were interleukin-8 (IL-8; 104.9% signal intensity compared to reference spots), platelet factor 4 (PF4; 98.5%), serpin E1 (97.2%), serpin F1 (96.5%), tissue inhibitor of metalloproteinase 1 (TIMP-1; 96.2%), and angiogenin (95.8%).

### Example 6: In-vitro wound healing and angiogenesis assay

To investigate whether L-EV-Mreg are able to positively influence wound healing and angiogenesis, scratch as well as tube formation assays using human endothelial cells (HUVEC) were performed. For this, human umbilical vein endothelial cells (HUVEC) were isolated from umbilical cords as described previously [28]. The cells were cultured in endothelial cell growth medium ECGM (PromoCell, Heidelberg, Germany) supplemented with 4 µL/mL of endothelial cell growth supplement, 0.1 ng/mL epidermal growth factor, 1 ng/mL basic fibroblast growth factor, 90 µg/mL heparin, 1 µg/mL hydrocortisone (all from PromoCell) and 10% fetal bovine serum (Thermo Fisher, Dreieich, Germany). HUVEC were maintained in a humidified atmosphere (5% carbon dioxide/95% air) at 37°C.

For the in-vitro wound healing assays ("scratch assays"), 15.000 HUVEC cell/cm² were seeded and grown until confluency. The cell monolayers were scratched to generate an in-vitro wound and maintained further in culture in the presence or absence of 1×10⁶ L-EV-Mreg/ml. After 8h (T8h), the size of the remaining cell-free gap was evaluated and compared to the size of the gap at T0h employing the ImageJ 1.41 software (NIH). For in-vitro tube formation assays, HUVEC were seeded in special cell culture dishes (Ibidi GmbH, Munich, Germany) according to the protocol provided by the manufacturer. Briefly, 10.000 HUVEC cells were seeded on Matrigel^{™} pre-coated wells with culture medium. After 1h, the cells were stimulated with or without 1×10⁶ L-EV-Mreg/ml. Photomicrographs of HUVEC were taken after 8h of culture, and tube formation as well as, angiogenesis related parameters were analyzed using the angiogenesis analyzer tool of the ImageJ software 1.41 (NIH) [25].

Results: Scratch assays revealed that the presence of L-EV-Mreg resulted in a slightly higher percentage of endothelial cell covered area compared to control, indicating positive effects of L-EV-Mreg on wound healing (L-EV-Mreg: 86.64±11.67%; control without L-EV-Mreg: 78.65±8.58%; ratio L-EV/control: 1.10±0.08%; P<0.05; Figure 6A and B). Tube formation assays showed that the presence of L-EV-Mreg during the culture period significantly influenced 6 out of the 18 investigated and angiogenesis-associated parameters (total of segment length: +20.70±2.98%; P<0.05; number of isolated segments: -6.10±2.78%; P<0.05; number of segments: +23.00±6.65%; P<0.05; total of master segment length: +17.50±1.84%; P<0.01; number of master segments: +33.10±7.00%; P<0.05; number of meshes:+ 41.30±7.33%; P<0.05; Figure 6C and D)

### Figure 7: Inhibition of T cell activation and expansion

To investigate the activation and expansion of T cells by Mreg and L-EV-Mreg, lymphocytes purified by Ficoll gradient centrifugation were activated by CD3/CD28 beads and subsequently incubated with Mreg cells or different concentrations of L-EV-Mreg. The activation system from ThermoFisher (Dynabeads^{™} human T-Activator CD3/CD28; #11131D) consists of uniform beads with a diameter of 4.5 µm. These beads are inert and superparamagnetic. They resemble antigen-presenting cells in size and are covalently coupled to anti-CD3 and anti-CD28 antibodies. These two antibodies provide primary and co-stimulatory signals optimized for effective activation and expansion of T cells. The ability of Mreg and L-EV-Mreg to regulate T cell activation and expansion was judged by the percentage of granzyme B positive lymphocytes. The corresponding analyses were performed flow cytometrically using a MACS Q10^{™} cytometer (Miltenyi) and the corresponding antibody from Invitrogen (#12-8899-41).

Results: The results are shown in Figure 7. Activation of the lymphocyte population by administration of CD3/CD28 beads results in a granzyme B positive cell population (control = 100%). Addition of Mreg to activated lymphocytes (ratio Mreg/lymphocytes = 1.34) leads to a significant reduction in the amount of granzyme B positive cells (20.78 ± 4.61). A similar effect is also obtained by the application of L-EV-Mreg, wherein the inhibition of T-cell activation (granzyme B positive cells) is dose-dependent and an effect comparable (25.85 ± 7.04) to that induced by the Mreg can be achieved by the administration of 3.2 million L-EV-Mreg/ml (ratio L-EV Mreg/lymphocytes = 9.14).

### LITERATURE

[1] Geissler EK, Hutchinson JA. Cell therapy as a strategy to minimize maintenance immunosuppression in solid organ transplant recipients. Curr Opin Organ Transplant 2013; 18: 408-15.
[2] Tang Q, Bluestone JA, Kang SM. CD4(+) Foxp3(+) regulatory T cell therapy in transplantation. J Mol Cell Biol 2012; 4: 11-21.
[3] Moreau A, Varey E, Bouchet-Delbos L, et al. Cell therapy using tolerogenic dendritic cells in transplantation. Transplant Res 2012; 1: 13.
[4] Broichhausen C, Riquelme P, Geissler EK, et al. Regulatory macrophages as therapeutic targets and therapeutic agent in solid organ transplantation. Curr Opin Organ Transplant 2012; 17: 332-42.
[5] Hutchinson JA, Riquelme P, Sawitzki B, et al. Cutting edge: immunological consequences and trafficking of human regulatory macrophages administered to renal transplant recipients. J Immunol 2011; 187: 2072-8.
[6] Hutchinson JA, Riquelme P, Brem-Exner BG, et al. Tranplant acceptance-inducing cells as an immune-conditioning therapy in renal transplantation. Transpl Int 2008; 21: 728-41.
[7] Hutchinson JA, Brem-Exner BG, Riquelme P, et al. A cell-based approach to the minimi-zation of immunosuppression in renal transplantation. Transpl Int 2008; 21: 742-54.
[8] Hutchinson JA, Roelen D, Riquelme P, et al. Preoperative treatment of a pre-sensitized kidney transplant recipient with donor-derived transplant acceptance-inducing cells. Transpl Int 2008; 21: 808-13.
[9] Hutchinson JA, Govert F, Riquelme P, et al. Administration of donor-derived transplant acceptance-inducing cells to the recipients of renal transplants from deceased donors is technically feasible. Clin Transplant 2009; 23: 140-5.
[10] Wang X, Jiang L, Liu Q: miR-18a-5p derived from mesenchymal stem cells-extracellular vesicles inhibits ovarian cancer cell proliferation, migration, invasion, and chemotherapy resistance. J Transl Med 2022, 20:258.
[11] Sun IO, Bae YU, Lee H, Kim H, Jeon JS, Noh H, Choi JS, Doh KO, Kwon SH: Circulating miRNAs in extracellular vesicles related to treatment response in patients with idiopathic membranous nephropathy. J Transl Med 2022, 20:224.
[12] Paolicelli RC, Bergamini G, Rajendran L: Cell-to-cell Communication by Extracellular Vesicles: Focus on Microglia. Neuroscience 2019, 405:148-157.
[13] Lee Y, Kim JH: The emerging roles of extracellular vesicles as intercellular messengers in liver physiology and pathology. Clin Mol Hepatol 2022, 28:706-724.
[14] Szwedowicz U, Lapinska Z, Gajewska-Naryniecka A, Choromanska A: Exosomes and Other Extracellular Vesicles with High Therapeutic Potential: Their Applications in Oncology, Neurology, and Dermatology. Molecules 2022, 27.
[15] Hummitzsch L, Zitta K, Rusch R, Cremer J, Steinfath M, Gross J, Fandrich F, Berndt R, Albrecht M: Characterization of the Angiogenic Potential of Human Regulatory Macrophages (Mreg) after Ischemia/Reperfusion Injury In Vitro. Stem Cells Int 2019, 2019:3725863.
[16] Sheng J, Xu Z: Three decades of research on angiogenin: a review and perspective. Acta Biochim Biophys Sin (Shanghai) 2016, 48:399-410.
[17] Medina RJ, O'Neill CL, O'Doherty TM, Knott H, Guduric-Fuchs J, Gardiner TA, Stitt AW: Myeloid angiogenic cells act as alternative M2 macrophages and modulate angiogenesis through interleukin-8. Mol Med 2011, 17:1045-1055.
[18] Bikfalvi A, Gimenez-Gallego G: The control of angiogenesis and tumor invasion by platelet factor-4 and platelet factor-4-derived molecules. Semin Thromb Hemost 2004, 30:137-144.
[19] Stefansson S, McMahon GA, Petitclerc E, Lawrence DA: Plasminogen activator inhibitor-1 in tumor growth, angiogenesis and vascular remodeling. Curr Pharm Des 2003, 9:1545-1564.
[20] He X, Cheng R, Benyajati S, Ma JX: PEDF and its roles in physiological and pathological conditions: implication in diabetic and hypoxia-induced angiogenic diseases. Clin Sci (Lond) 2015, 128:805-823.
[21] Chirco R, Liu XW, Jung KK, Kim HR: Novel functions of TIMPs in cell signaling. Cancer Metastasis Rev 2006, 25:99-113.
[22] Albini A, Gallazzi M, Palano MT, Carlini V, Ricotta R, Bruno A, Stetler-Stevenson WG, Noonan DM: TIMP1 and TIMP2 Downregulate TGFbeta Induced Decidual-like Phenotype in Natural Killer Cells. Cancers (Basel) 2021, 13.
[23] Cao Y, Linden P, Farnebo J, Cao R, Eriksson A, Kumar V, Qi JH, Claesson-Welsh L, Alitalos K, Proc. Natl. Acad. Sci. USA 1998; 95: 14389-14394.
[24] Yanez-Mo M, Siljander PR, Andreu Z, Zavec AB, Borras FE, Buzas EI, Buzas K, Casal E, Cappello F, Carvalho J, et al: Biological properties of extracellular vesicles and their physiological functions. J Extracell Vesicles 2015, 4:27066.
[25] Thery C, Witwer KW, Aikawa E, Alcaraz MJ, Anderson JD, Andriantsitohaina R, Antoniou A, Arab T, Archer F, Atkin-Smith GK, et al: Minimal information for studies of extracellular vesicles 2018 (MISEV2018): a position statement of the International Society for Extracellular Vesicles and update of the MISEV2014 guidelines. J Extracell Vesicles 2018, 7:1535750.
[26] Witwer KW, Goberdhan DC, O'Driscoll L, Thery C, Welsh JA, Blenkiron C, Buzas EI, Di Vizio D, Erdbrugger U, Falcon-Perez JM, et al: Updating MISEV: Evolving the minimal requirements for studies of extracellular vesicles. J Extracell Vesicles 2021, 10:e12182.
[27] Gao W, Guo X, Wang Y, Jian D, Li M: Monocyte-derived extracellular vesicles upon treated by palmitate promote endothelial migration and monocytes attachment to endothelial cells. Biochem Biophys Res Commun 2020, 523:685-691.
[28] Baudin B, Bruneel A, Bosselut N, Vaubourdolle M: A protocol for isolation and culture of human umbilical vein endothelial cells. Nat Protoc 2007, 2:481-485.

## Claims

1. Macrophage-derived extracellular vesicle having a size between 0.1-10 µm, wherein said vesicle exerts an immunoregulatory, angiogenic, anti-inflammatory and/or tissue regenerative properties activity.

2. Macrophage-derived extracellular vesicle of claim 1, wherein said vesicle comprises one or more markers selected from the group consisting of CD11c, CD86, CD31 and CD45 on its surface.

3. Macrophage-derived extracellular vesicle of claim 1 or 2, wherein said vesicle comprises one or more markers selected from the group consisting of LAMP-1, CD9, CD63 and CD81 on its surface.

4. Macrophage-derived extracellular vesicle of any of claims 1-3, wherein said vesicle is derived from an immunoregulatory macrophage (Mreg), and preferably from an immunoregulatory macrophage which expresses the markers CD258, DHRS9 and IDO.

5. Macrophage-derived extracellular vesicle of any of claims 1-4, wherein said vesicle comprises one or more proteins selected from the group of interleukin-8, platelet factor-4, serpin E1, serpin F1, TIMP-1, and angiogenin.

6. Macrophage-derived extracellular vesicle of claim 5, wherein said vesicle interleukin-8, platelet factor-4, serpin E1, serpin F1, TIMP-1, and angiogenin.

7. Macrophage-derived extracellular vesicle of any of claims 1-6, wherein said vesicle does not contain a nucleus.

8. Macrophage-derived extracellular vesicle of any of claims 1-7, wherein said vesicle does not contain any cell organelle selected from the group of nucleus, mitochondria, endoplasmatic reticulum and Golgi complex.

9. Macrophage-derived extracellular vesicle of any of claims 1-8, obtainable by a method which includes the differentiation of CD14 positive blood monocytes into immunoregulatory macrophages by culturing the monocytes in a culture medium in the presence of (i) M-CSF and/or GM-CSF, (ii) a CD16 ligand, and (iii) IFN-y and subsequently obtaining the extracellular vesicles from the culture medium.

10. Pharmaceutical composition comprising the macrophage-derived extracellular vesicle of any of claims 1-9.

11. Macrophage-derived extracellular vesicle of any of claims 1-9 or pharmaceutical composition of claim 10 for use in medicine.

12. Macrophage-derived extracellular vesicle of any of claims 1-9 or pharmaceutical composition of claim 10 for use in a method of suppressing transplant rejection and/or prolonging transplant survival in a subject receiving a transplant.

13. Macrophage-derived extracellular vesicle or pharmaceutical composition for use in a method of claim 12, wherein said transplant is an allogeneic transplant.

14. Macrophage-derived extracellular vesicle of any of claims 1-9 or pharmaceutical composition of claim 10 for use in a method of promoting or sustaining the engraftment or effect of regulatory T cell cell-based medicinal products.

15. Macrophage-derived extracellular vesicle of any of claims 1-9 or pharmaceutical composition of claim 10 for use in a method of treating or preventing an autoimmune disease, an inflammatory disease, or a hypersensitivity reaction.

16. Macrophage-derived extracellular vesicle or pharmaceutical composition for use in a method of claim 15, wherein said autoimmune disease is selected from the group consisting of systemic lupus erythematosus (SLE), scleroderma, Sjögren's syndrome, polymyositis, dermatomyositis, and other systemic autoimmune conditions; rheumatoid arthritis (RA), juvenile rheumatoid arthritis, and other inflammatory arthritides; ulcerative colitis, Crohn's disease, and other inflammatory bowel diseases; autoimmune hepatitis, primary biliary cirrhosis, and other autoimmune liver diseases; cutaneous small-vessel vasculitis, granulomatosis with polyangiitis, eosinophilic granulomatosis with polyangiitis, Behcet's disease, thromboangiitis obliterans, Kawasaki disease, and other large-, medium- or small-vessel vasculitides of autoimmune aetiology; Multiple sclerosis (MS) and neuroimmunological disorders; Type I diabetes, autoimmune thyroid dysfunction, autoimmune pituitary dysfunction, and other autoimmune endocrinological disorders; haemolytic anaemia, thrombocytopaenic purpura and other autoimmune disorders of the blood and bone marrow; psoriasis, pemphigus vulgaris, pemphigoid and other autoimmune dermatological conditions.

17. Macrophage-derived extracellular vesicle or pharmaceutical composition for use in a method of claim 15, wherein said inflammatory disease is selected from the group consisting of arterial occlusive diseases, such as peripheral artery occlusive disease (pAOD), critical limb ischaemia, arteriosclerosis, cerebral infarction, myocardial infarction, renal infarction, intestinal infarction, angina pectoris, and other conditions caused by arterial occlusion or constriction; microvascular angina, also known as cardiac syndrome X; inflammation associated systemic with metabolic disorders, including Type II diabetes and obesity-related metabolic syndrome; dermatological diseases, including eczema.

18. Macrophage-derived extracellular vesicle or pharmaceutical composition for use in a method of claim 15, wherein said hypersensitivity reaction is selected from the group of asthma, eczema, allergic rhinitis, angioedema, drug hypersensitivity and mastocytosis.

19. Macrophage-derived extracellular vesicle of any of claims 1-9 or pharmaceutical composition of claim 10 for use in a method of inducing wound healing or promoting tissue-repair processes by participating in tissue remodelling, tissue regeneration, angiogenesis, vasculogenesis, or prevention/limitation of fibrosis.

20. Macrophage-derived extracellular vesicle of any of claims 1-9 or pharmaceutical composition of claim 10 for use in a method of treating micro- or macroangiopathies of the lower limbs in a subject.

21. Macrophage-derived extracellular vesicle or pharmaceutical composition for use in a method of claim 20, wherein said macroangiopathy is selected from the group consisting of aneurysm, dissection, atherosclerosis, atherothrombosis, peripheral arterial occlusive disease (PAD), claudicatio intermittens, necrosis and gangrene, vascular malformation, Leriche syndrome, or compression syndrome.

22. Macrophage-derived extracellular vesicle or pharmaceutical composition for use in a method of claim 20, wherein said microangiopathy is selected from the group consisting of angiitis, arteritis, angiodysplasia, atrophy blanche, dermatosclerosis, Determann syndrome, diabetic angiopathy, endangiitis obliterans, erythromelalgia, fibro muscular dysplasia, malum perforans, Mönckeberg Sclerosis, Osler's disease, compartment syndrome, Paget-von-Schroetter syndrome, Raynaud's syndrome, and leg ulcers.

23. Macrophage-derived extracellular vesicle or pharmaceutical composition for use in a method of claim 22, wherein said leg ulcers are diabetic or venous leg ulcers.

24. Macrophage-derived extracellular vesicle or pharmaceutical composition for use in a method of claim 23, wherein said method comprises the administration of vesicles directly into the ulcer by subcutaneous or intramuscular injection.

25. Macrophage-derived extracellular vesicle or pharmaceutical composition for use in a method of any of claims 20-24, wherein said micro- or macroangiopathies are diabetic micro- or macroangiopathies.

26. A process for preparing macrophage-derived extracellular vesicles having immunoregulatory activity, said process comprising:
(a) isolating CD14 positive monocytes from a blood sample of a subject;
(b) culturing the monocytes in a culture medium containing (i) M-CSF and/or GM-CSF, and (ii) a CD16 ligand;
(c) contacting the cells with IFN-y; and
(d) obtaining the macrophage-derived extracellular vesicles from the culture medium.

27. Process of claim 26, wherein the culture medium in step (b) comprises human blood serum, such as human AB serum.

28. Process of claim 26 or 27, wherein the concentration of M-CSF and/or GM-CSF in step (b) is in the range of 5-100 ng/ml, preferably 20-25 ng/ml.

29. Process of any of claims 26-28, wherein the monocytes in step (b) are cultured for at least 3 days, for at least 4 days, for at least 5 days, for at least 6, or for at least 7 days prior to IFN-y stimulation.

30. Process of any of claims 26-29, wherein the culturing in step (b) is performed in a gas-permeable bag which is preferably made of plastic.

31. Process of any of claims 26-30, wherein the concentration of IFN-y in step (c) is in the range of 5-100 ng/ml, preferably 20-25 ng/ml.

32. Process of any of claims 26-31, wherein the macrophage-derived extracellular vesicles are obtained from the culture medium in step (d) by centrifugation.

33. Process of claim 32, wherein the macrophage-derived extracellular vesicles in step (d) are separated from macrophage cells by centrifugation.

34. A process for preparing an immunoregulatory T cell, said process comprising:
(a) obtaining T cells of a subjects;
(b) co-culturing the T cells with an macrophage-derived extracellular vesicle of any of claims 1-9;
(c) obtaining the immunoregulatory T cell from the culture medium.
